# DEMANDE DE BREVET EUROPEEN

(11) **EP 3 124 011 A1**
(43) Date de publication de la demande: **01.02.2017**
(21) Numéro de dépôt: 16181342.3
(22) Date de dépôt: 26.07.2016
(51) Int. Cl.: A61K 8/34, A61K 8/37, A61Q 19/00, A61K 8/81, A61K 8/92, A61K 8/06

(54) **BASE DE FORMULATION COSMETIQUE CONCENTREE**

(30) Priorité: 28.07.2015 FR 1557196
(71) Demandeur: Laboratoires M & L, 04100 Manosque (FR)
(72) Inventeur: MILLET, Magali, 04190 LES MEES (FR); PERRIN, Mélodie, 04700 ORAISON (FR)
(74) Mandataire: Novagraaf Technologies

(57) **Abrégé**

La présente invention se rapporte à une base de formulation cosmétique concentrée comprenant une phase aqueuse glycérinée comprenant de 15 à 50 % en poids de glycérine, de 2 à 50% en poids d'eau, et de 0,2 à 3% en poids de gélifiants par rapport au poids total de la composition, et une phase huileuse comprenant de 20 à 60% en poids d'émollients. La présente invention se rapporte également à une composition cosmétique comprenant ladite base de formulation ainsi qu'à l'utilisation cosmétique de ladite composition cosmétique.

## Description

### Domaine technique

La présente invention se rapporte à une base de formulation cosmétique concentrée comprenant une phase aqueuse glycérinée comprenant de 15 à 50 % en poids de glycérine, de 2 à 50% en poids d'eau, et de 0,2 à 3% en poids de gélifiants par rapport au poids total de la composition, et une phase huileuse comprenant de 20 à 60% en poids d'émollients.

La présente invention se rapporte également à une composition cosmétique comprenant ladite base de formulation ainsi qu'à l'utilisation cosmétique d'une composition cosmétique selon l'invention.

La présente invention trouve une application notamment dans les domaines cosmétique et dermatologique.

Dans la description ci-dessous, les références entre crochets ([ ]) renvoient à la liste des références présentée à la fin du texte.

### Etat de la technique

Il existe actuellement de nombreuses formes différentes de compositions cosmétiques quelque soit leur application cosmétique. Les compositions cosmétiques comprennent généralement des actifs cosmétiques inclus dans des compositions, par exemple des huiles, crèmes, émulsions, des lotions etc. Ces compositions peuvent être qualifiées de « bases cosmétiques ». Il existe actuellement de nombreuses bases cosmétiques à partir desquelles les compositions finies incluant les actifs cosmétiques sont obtenues.

Toutefois, pour obtenir les compositions finies, il est nécessaire de mettre en oeuvre des procédés complexes pouvant impliquer optionnellement des dilutions et agitations à chaud, c'est-à-dire en chauffant la base, pour inclusion du ou des actifs cosmétiques.

Il existe également des bases cosmétiques adaptées pour l'obtention de composition sous la forme d'émulsion. Par ailleurs, les bases cosmétiques connues peuvent présenter des temps de conservations courts et/ou inadaptés à la production et conservation industrielle desdites bases. Aussi, la préparation desdites bases doit être faite au coup par coup réduisant ainsi la flexibilité de production. Aussi, afin d'éviter une dégradation dans le temps et/ou une contamination/colonisation des bases, par exemple dû à la présence dans les bases d'agents extérieurs indésirables, il est parfois nécessaire d'incorporer des conservateurs, par exemple de l'alcool, susceptibles d'altérer la formulation/galénique et/ou la composition des bases cosmétiques.

Il existe donc un réel besoin de trouver de nouvelles bases de produits cosmétiques, en particulier de nouvelles bases cosmétiques permettant l'incorporation d'actifs cosmétiques sans mise en oeuvre de procédés complexes.

Il existe également un réel besoin de trouver de nouvelles bases cosmétiques présentant une texture adaptée quelle que soit leur utilisation et des propriétés de conservations adaptées.

En outre, il existe également un réel besoin de trouver de nouvelles bases cosmétiques par exemple sans conservateur, avec des coûts de fabrication réduits et dont la réalisation industrielle ne soit pas un obstacle.

Enfin, il existe un réel besoin de trouver de nouvelles bases cosmétiques permettant l'incorporation d'actifs cosmétiques via la mise en oeuvre de procédés simples.

### Description de l'invention

La présente invention a précisément pour but de répondre à ces nombreux besoins en fournissant une base concentrée de formulation cosmétique comprenant :
- une phase aqueuse glycérinée comprenant de 15 à 50 % en poids de glycérine, de 2 à 50% en poids d'eau, et de 0,2 à 3% en poids d'au moins un gélifiant par rapport au poids total de la base,
- une phase huileuse comprenant de 20 à 60% en poids d'au moins un émollient, de 2 à 10% en poids d'au moins un émulsionnant par rapport au poids total de la base.

Les inventeurs de la présente sont en effet les touts premiers à avoir mis en évidence, de manière tout à fait inattendue, qu'une base de formulation cosmétique comprenant une phase aqueuse glycérinée comprenant de 15 à 50 % en poids de glycérine, de 2 à 50% en poids d'eau, et de 0,2 à 3% en poids d'au moins un gélifiant par rapport au poids total de la base, et une phase huileuse comprenant de 20 à 60% en poids d'au moins un émollient, de 2 à 10% en poids d'au moins un émulsionnant par rapport au poids total de la base permet avantageusement l'obtention d'une base concentrée de formulation cosmétique permettant l'obtention de composition cosmétique par simple dilution et/ou mélange de la base avec une solution.

En particulier, les inventeurs ont mis en évidence de manière tout à fait inattendue que la base cosmétique selon l'invention permet l'obtention de compositions cosmétiques sans étape de chauffage. En d'autres termes, les inventeurs ont mis en évidence que la base cosmétique selon l'invention permet avantageusement l'obtention de compositions cosmétiques sans mise en oeuvre de procédés complexes nécessitant, par exemple un chauffage de la base cosmétique.

Les inventeurs de la présente ont également mis en évidence, de manière tout à fait inattendue que la base cosmétique concentrée selon l'invention est avantageusement microbiologiquement plus stable et permet de part la concentration de ces différents composants l'obtention d'un grand nombre de compositions cosmétiques avec différentes textures.

En outre, les inventeurs ont également démontré et obtenu, notamment dû au choix des ingrédients, une base concentrée avec une faible teneur en eau, une forte teneur en émollients qui est stable dans le temps que ce soit au niveau physicochimique et/ou microbiologique.

Les inventeurs ont démontré de manière surprenante que la base de formulation, notamment de part ces propriétés de conservation et/ou de stabilité, peut être avantageusement stockée sans dégradation. En outre, la base de formulation peut être utilisée, par exemple mélangée avec une solution afin de former une composition cosmétique immédiate, sans nécessité de procédé de fabrication particulier, par exemple simple mélange sans chauffage. Avantageusement, les inventeurs ont également démontré que la base de formulation permet d'obtenir des compositions cosmétiques fraiches, permettant de diminuer l'altération éventuelle du ou des actifs cosmétiques. En outre, les inventeurs ont démontré, de part la simplicité de fabrication de la composition à partir de la base de formulation une fabrication « à la maison » d'une composition cosmétique à partir de cette base.

En outre, les inventeurs ont démontré de manière surprenante que le choix des gammes de concentrations, notamment concernant l'eau et la glycérine, permet avantageusement et contrairement au base connue, d'avoir une base de formulation cosmétique stable, tant au niveau physicochimique que microbiologique.

De plus, les inventeurs ont également démontré, par exemple lorsque la concentration en eau de la base cosmétique est basse, par exemple comprise de 2 à 50%, par exemple de 2 à 35%, la base cosmétique selon l'invention peut être avantageusement diluée à l'utilisation, par exemple fabrication « à la maison », par exemple par le consommateur, par exemple avec de l'eau, par exemple de l'eau florale, et permet avantageusement l'obtention d'une composition avec une sensorialité et une viscosité adaptée à une utilisation cosmétique. En d'autres termes, la base cosmétique selon l'invention permet avantageusement l'obtention de compositions cosmétiques microbiologiquement stables avec une sensorialité et une viscosité adaptées à une utilisation cosmétique par simple mélange.

En outre, les inventeurs, contrairement à l'enseignement de l'art, ont obtenu une base cosmétique stable notamment via une sélection particulière de gammes de concentrations et de choix de composants. En particulier, les inventeurs de par la sélection de gammes de concentrations et des ingrédients, notamment l'eau, la glycérine, au moins un émulsionnant, et au moins un gélifiant a permis l'obtention d'une base cosmétique qui permet avantageusement par simple mélange à froid, par exemple par dilution à froid, l'obtention d'une composition cosmétique stable avec une viscosité adaptée à une utilisation cosmétique.

De plus, les inventeurs, contrairement à l'enseignement de l'art, ont obtenu une base cosmétique stable notamment via une sélection particulière de phase aqueuse et de phase huileuse comprenant chacune indépendamment des ingrédients particuliers et des gammes de concentrations desdits ingrédients.

Dans la présente, par « base de formulation cosmétique» on entend une base utilisable pour la fabrication de compositions cosmétiques. Il peut s'agir par exemple d'une base pour la formation de toute forme de composition cosmétique connue de l'homme du métier. Il peut s'agir par exemple d'une base de formulation cosmétique permettant par exemple l'obtention d'une crème, par exemple une émulsion huile dans l'eau, d'une lotion, d'un lait.

Dans la présente, par « base concentrée de formulation cosmétique» on entend une base de formulation cosmétique qui permet l'obtention d'une composition cosmétique, par exemple, dilution de ladite base par une solution, par exemple une solution aqueuse, une solution huileuse, une solution hydroalcoolique. Il peut s'agir par exemple d'eau, de solvant comprenant au moins un actif cosmétique et/ou d'extrait comprenant au moins un actif cosmétique, quelque soit sa forme, et adapté pour son incorporation dans un produit cosmétique.

Dans la présente par « gélifiant » on entend tout gélifiant connu de l'homme du métier et/ou disponible dans le commerce susceptible d'être utilisé dans une base cosmétique. Il peut s'agir par exemple d'un gélifiant choisi dans le groupe comprenant les silicates, les celluloses, les acrylates, les dérivés d'algues, les gommes végétales, les pectines, les dérivés d'amidon, la gomme de xanthane, la gomme de sclérotium, une gomme naturelle et/ou issue de procédés biotechnologiques ou un mélange de ceux-ci.

Dans la présente par « silicates » on entend tout silicate connu de l'homme du métier susceptible d'être utilisé dans une base cosmétique. Il peut s'agir par exemple de silicates disponibles dans le commerce susceptibles d'être utilisés dans une base cosmétique. Il peut s'agir par exemple de la bentonite, l'hectorite, la montmorillonite, le sodium magnésium silicate, le magnésium aluminium silicate.

Dans la présente par « cellulose » on entend toute cellulose et/ou dérivés de cellulose connus de l'homme du métier susceptible d'être utilisés dans une base cosmétique. Il peut s'agir par exemple de cellulose et/ou dérivés de cellulose disponibles dans le commerce. Il peut s'agir par exemple de microcristalline cellulose, cellulose gum, carboxymethyl cellulose, cétyl hydroxyéthylcellulose, éthylcellulose, hydroxybutyl méthylcellulose, hydroxypropylcellulose, gomme de cellulose hydrolysée, hydroxypropylméthylcellulose, hydroxyéthylcellulose ou un quelconque mélange de ceux-ci.

Dans la présente par « acrylates » on entend tout acrylate connu de l'homme du métier et/ou disponible dans le commerce susceptible d'être utilisé dans une base cosmétique. Il peut s'agir par exemple l'acrylates/Beheneth-25 methacrylate copolymer, l'acrylates/C10-30 alkyl acrylate crosspolymer, le sodium polyacrylate starch, sodium polyacrylate, l'hydroxyethyl acrylate / sodium acryloyldimethyl taurate copolymère, dérivés d'Acrylates Crosspolymer, les carbomers, polyacrylamides et leurs dérivés ou un quelconque mélange de ceux-ci.

Dans la présente par « dérivé d'algues » on entend tout dérivé d'algues connu de l'homme du métier et/ou disponible dans le commerce susceptible d'être utilisé dans une base cosmétique. Il peut s'agir par exemple de carraghénanes, d'agar, alginates.

Dans la présente par « gommes végétales » on entend toute gomme végétale et/ou toute gomme naturelle connue de l'homme du métier et/ou disponible dans le commerce susceptible d'être utilisée dans une base cosmétique. Il peut s'agir par exemple de gomme de caroube, de gomme guar, de gomme de tara, de gomme d'acacia du Sénégal, gomme arabique, gomme adragante, gomme de tamarin.

Dans la présente par « gomme issue d'un procédé biotechnologique » on entend toute gomme d'un procédé biotechnologique connu de l'homme du métier et/ou disponible dans le commerce susceptible d'être utilisée dans une base cosmétique. Il peut s'agir par exemple d'une gomme végétale et/ou naturelle obtenue à partir d'un procédé biotechnologique. Il peut s'agir par exemple d'une gomme de xanthane, d'une gomme de dehydroxanthane, d'une gomme de sclérotium, d'une gomme gellane ou un quelconque mélange de ceux-ci.

Dans la présente par « pectine » on entend tout polymère de polysaccharides acides à savoir d'acide polygalacturonique issu de végétaux connu de l'homme du métier et/ou disponible dans le commerce susceptible d'être utilisé dans une base cosmétique. Il peut s'agir par exemple de pectine issue de pépins et/ou de zestes, par exemple de groseilles, de pommes, de coings et/ou d'agrumes.

Dans la présente par « dérivés d'amidon » on entend tout dérivé d'amidon connu de l'homme du métier et/ou disponible dans le commerce susceptible d'être utilisé dans une base cosmétique. Il peut s'agir par exemple du sodium carboxymethyl starch, l'hydroxypropyl starch phosphate, de l'amidon de tapioca, de l'amidon de tapioca copolymère (« Tapioca Starch Crosspolymer »), d'amidon de maïs, d'amidon modifié de pomme de terre, Phosphate de diamidon, d'amidon de riz dimethylimidazolidinone, d'amidon de riz, d'amidon de maïs cireux pre-gélatinisé ou un quelconque mélange de ceux-ci.

Avantageusement le gélifiant peut être choisi dans le groupe comprenant la xanthane, la déhydroxanthane, la gomme guar, la gomme de caroube, le sodium polyacrylate et le l'hydroxyethyl acrylate / sodium acryloyldimethyl taurate copolymer ou n'importe quel mélange de ceux-ci.

Avantageusement, le gélifiant est facilement hydratable, permettant de produire une viscosité importante, par exemple une viscosité supérieure à 20 000 cps, par exemple de 20000, 30000, 40000 ou 50000 cps, de façon instantanée lors d'un mélange à froid de la base concentrée avec une solution telle que décrite ci-dessus. Dans la présente, la mesure de la viscosité peut être réalisée par tout procédé ou dispositif connu de l'homme du métier, par exemple avec un dispositif brookfield RV6V12.

Avantageusement, la base concentrée peut comprendre au moins un, ou au moins deux gélifiants.

Dans la présente, ledit au moins un gélifiant peut être présent dans la base de formulation cosmétique à une concentration comprise de 0,2 à 3% en poids par rapport au poids total de ladite base de formulation cosmétique, par exemple de 0,3 à 1,5% en poids par rapport au poids total de ladite base de formulation.

Avantageusement, lorsque le gélifiant comprend une gomme issue d'un procédé biotechnologique et/ou une gomme naturelle, ladite gomme peut être présente dans la base de formulation cosmétique à une concentration comprise de 0,1 à 0,5% en poids par rapport au poids total de ladite base de formulation.

Avantageusement, lorsque le gélifiant comprend au moins un acrylate, ledit au moins un acrylate peut être présent dans la base de formulation cosmétique à une concentration comprise de 0,4 à 1,5% en poids par rapport au poids total de ladite base de formulation.

Dans la présente, l'eau peut être de l'eau minérale, de l'eau distillée, de l'eau osmosée, de l'eau florale et/ou provenant d'un extrait aqueux.

Dans la présente, l'eau peut être présente dans la base de formulation cosmétique à une concentration comprise de 2 à 50% en poids par rapport au poids total de ladite base de formulation cosmétique, par exemple de 5 à 45% en poids par rapport au poids total de ladite base de formulation, de 5 à 35% en poids par rapport au poids total de ladite base de formulation cosmétique.

Dans la présente, lorsque l'eau provient d'un extrait aqueux, l'eau provenant dudit extrait aqueux peut être présente dans la base de formulation cosmétique à une concentration comprise de 2 à 50% en poids par rapport au poids total de ladite base de formulation cosmétique, par exemple de 5 à 45% en poids par rapport au poids total de ladite base de formulation, par exemple de 2 à 35%, par exemple de 5 à 35% en poids par rapport au poids total de ladite base de formulation cosmétique.

Dans la présente par glycérine on entend toute glycérine connue de l'homme du métier et/ou disponible dans le commerce susceptible d'être utilisée dans une base cosmétique. Il peut s'agir par exemple de la glycérine commercialisée par la société Univar sous la référence commerciale Pharma glycerin vegetable.

Dans la présente, la glycérine peut être présente dans la base de formulation cosmétique à une concentration comprise de 15 à 50% en poids par rapport au poids total de ladite base de formulation cosmétique, par exemple de 25 à 45% en poids par rapport au poids total de ladite base de formulation.

Dans la présente, la phase huileuse de la base de formulation concentrée cosmétique peut comprendre en outre au moins un alcool gras.

Dans la présente par « alcool gras » on entend tout alcool gras connue de l'homme du métier et/ou disponible dans le commerce susceptible d'être utilisé dans une base cosmétique. Il peut s'agir par exemple d'un alcool gras choisi dans le groupe comprenant le cétyl alcool, le behenyl alcool, le cétearyl alcool, le decyl alcool, l'isocetyl alcool, l'isopropyl alcool, l'isostearyl alcool, le lauryl alcool, le myristyl alcool ou un quelconque mélange de ceux-ci

Dans la présente, la base concentrée peut comprendre au moins un, au moins deux, au moins trois, au moins quatre alcools gras.

Dans la présente, l'alcool gras peut être présent dans la base concentrée de formulation cosmétique à une concentration comprise de 0,5 à 1,5% en poids par rapport au poids total de ladite base, par exemple de 0,7 à 1,25% en poids par rapport au poids total de ladite base.

Dans la présente par « émollient » on entend tout émollient connu de l'homme du métier et/ou disponible dans le commerce susceptible d'être utilisé dans une base cosmétique. Il peut s'agir par exemple d'un émollient choisi dans le groupe comprenant une huile végétale, un beurre végétal, un ester d'origine synthétique ou d'origine végétale ou un mélange de ceux-ci.

Dans la présente par « huile végétale » on entend toute huile végétale connue de l'homme du métier et/ou disponible dans le commerce susceptible d'être utilisée dans une base cosmétique. Il peut s'agir d'une huile végétale choisie dans le groupe comprenant une huile de tournesol, une huile de ricin, une huile d'onagre, une huile de jojoba, une huile de noix, une huile de coprah les huiles de germe de blé, de tournesol, d'argan, d'hibiscus, de coriandre, de pépins de raisin, de sésame, de maïs, d'abricot, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de lavande, de bourrache, de millet, d'orge, de quinoa, de seigle, de carthame, de bancoulier, de passiflore, de rosier muscat ou de camélia ou un mélange de celles-ci.

Dans la présente par « beurre végétal » on entend tout beurre végétal connu de l'homme du métier et/ou disponible dans le commerce susceptible d'être utilisé dans une base cosmétique. Il peut s'agir d'un beurre végétal choisi dans le groupe comprenant un beurre d'amande, un beurre de karité, beurre de cacao, beurre d'olive, beurre d'argan, beurre de mangue, beurre de cupuaçu ou un mélange de ceux-ci.

Dans la présente par « ester d'origine synthétique ou végétal » on entend tout ester d'origine synthétique ou végétal connu de l'homme du métier et/ou disponible dans le commerce susceptible d'être utilisé dans une base cosmétique. Il peut s'agir par exemple d'esters d'acides et/ou de mono-alcool, par exemple des mono- et polyesters d'acides linéaires saturés en C₂-C₁₀, de préférence en C₆-C₁₀, de mono-alcools linéaires saturés en C₁₀-C₁₈, de préférence C₁₀-C₁₄, de mono- et polyesters d'acides linéaires saturés en C₁₀-C₂₀ et de mono-alcools ramifiés ou insaturés en C₃-C₂₀, de préférence en C₃-C₁₀; de mono- et polyesters d'acides ramifiés ou insaturés en C₅-C₂₀ et de mono-alcools ramifiés ou insaturés en C₅-C₂₀; de mono- et polyesters d'acides ramifiés ou insaturés en C₅-C₂₀ et de mono-alcools linéaires en C₂-C₄ ; de triglycérides d'acides gras en C₆-C₁₂, par exemple de triglycérides d'acides caprylique et caprique et la triheptanoïne; d'acides gras ramifiés et/ou insaturés en C₁₀-C₂₀, par exemple d'acides linoléique, d'acide laurique et/ou d'acide myristique ; d'alcools gras ramifiés et/ou insaturés en C₁₀-C₂₀, par exemple l'octyldodécanol, l'alcool oléylique; d'hydrocarbures, par exemple le squalane végétal extrait de l'huile d'olive ; de carbonates de dialkyle, par exemple le dicaprylyl carbonate, le diéthylhexyl carbonate ; le dialkyléthers, par exemple le dicaprylyl éther ; ou un quelconque mélange de ceux-ci.

Dans la présente, la base concentrée peut comprendre au moins un, au moins deux, au moins trois, au moins quatre émollient(s).

Dans la présente, l'émollient peut être présent dans la base de formulation cosmétique à une concentration comprise de 20 à 60% en poids par rapport au poids total de ladite base de formulation cosmétique, par exemple de 25 à 50% en poids par rapport au poids total de ladite base de formulation.

Dans la présente par « émulsionnant » on entend tout émulsionnant connu de l'homme du métier et/ou disponible dans le commerce susceptible d'être utilisé dans une base cosmétique. Il peut s'agir par exemple de monoester ou polyester de glycérol, de dérivés de lécithine, d'émulsionnants issus de la condensation d'un alcool gras, la famille des esters de sucres, les alcools gras, des acides gras, les dérivés d'acides aminés, les dérivés d'esters d'acide laurique, les dérivés d'esters d'acides stéariques, les dérivés d'esters d'acides phosphoriques, des esters d'acide citrique, des esters de polyéthylène glycol, des éthers de polyéthylène glycol, des sarcosinates, des dérivés de cires d'origine végétale ou animale ou un mélange de ceux-ci.

Dans la présente par monoester ou polyester de glycérol on entend tout monoester ou polyester de glycérol connu de l'homme du métier et/ou disponible dans le commerce susceptible d'être utilisé dans une base cosmétique. Il peut s'agir par exemple du glycéryl ester, du glyceryl stearate, PEG-20 glyceryl stearate, glyceryl oleate citrate, glyceryl stearate citrate, polyglyceryl-6-distearate, polyglyceryl-3-beeswax, polyglyceryl-3-diisostearate, polyglyceryl-10-stearate, polyglyceryl-10 dipalmitate, candelilla/jojoba/rice bran polyglyceryl-3-esters, polyglyceryl-3- rice branate, Polyglyceryl-3 MethylGlucose Distearate ou un mélange de ceux-ci.

Dans la présente par dérivé de lécithine on entend tout dérivé de lécithine connu de l'homme du métier et/ou disponible dans le commerce susceptible d'être utilisé dans une base cosmétique. Il peut s'agir par exemple la lécithine hydrogénée ou un mélange de celles-ci.

Dans la présente par émulsionnants issus de la condensation d'un alcool gras et d'un sucre on entend tout émulsionnant issus de la condensation d'un alcool gras et d'un sucre connu de l'homme du métier et/ou disponible dans le commerce susceptible d'être utilisé dans une base cosmétique. Il peut s'agir par exemple d'un émulsionnant issu de la condensation du sorbitol, du glucose, du saccharose, du fructose, xylitol et d'un alcool gras tel que décrit ci-dessus.

Dans la présente par « acides gras » on entend un acide carboxylique à chaîne aliphatique avec une chaîne carbonée de 4 à 22 atomes de carbone. Il peut s'agir par exemple d'un acide gras choisi dans le groupe comprenant l'acide caprylique, l'acide caprique, l'acide laurique, l'acide myristique, l'acide palmitique, l'acide stéarique, l'acide arachidique, l'acide dodécanoïque, l'acide myristoléique, l'acide palmitoléique, l'acide sapiénique, l'acide oléique, l'acide élaïdique, l'acide trans-vaccénique, l'acide linoléique, l'acide linolélaïdique, l'acide α-linolénique, l'acide γ-linolénique, l'acide dihomo-γ-linolénique, l'acide arachidonique, l'acide eicosapentaénoïque, l'acide clupanodonique ou un mélange de ceux-ci.

Dans la présente par « ester de sucre » on entend tout ester de sucre connu de l'homme du métier et/ou disponible dans le commerce susceptible d'être utilisé dans une base cosmétique. Il peut s'agir par exemple d'ester de sorbitol, d'ester de glucose, d'ester de saccharose, de xylitol, de fructose comme par exemple le sorbitan oleate, le sorbitan olivate, le sorbitan stearate, le coco-glucoside, l'arachidyl glucoside, le myristyl glucoside, l'hydroxystearyl glucoside, le C12-20 alkyl glucoside, le cetearyl glucoside, le PEG-20 methylglucose sesquistearate, le methylglucose sesquistearate, le sucrose cocoate, le sucrose palmitate, le sucrose laurate, le sucrose distearate, le sucrose stearate, le sucrose tristearate, le xylityl glucoside.

Dans la présente par « dérivé d'acides aminés » on entend tout dérivé d'acides aminés connu de l'homme du métier et/ou disponible dans le commerce susceptible d'être utilisé dans une base cosmétique. Il peut s'agir par exemple le sodium lauroyl glutamate, le sodium cocoyl glutamate, le sodium stearoyl lactylate, le sodium stearoyl glutamate ou un quelconque mélange de ceux-ci.

Dans la présente par « dérivés d'esters d'acide laurique » on entend un dérivé choisi dans le groupe comprenant le sucrose laurate, le glycéryl laurate et dérivés, le sucrose dilaurate, le polyglycéryl laurate et dérivés, le sorbitan laurate ou un quelconque mélange de ceux-ci.

Dans la présente par « dérivé d'ester d'acide stéarique » on entend tout dérivé d'ester d'acide stéarique connu de l'homme du métier et/ou disponible dans le commerce susceptible d'être utilisé dans une base cosmétique. Il peut s'agir par exemple d'un dérivé d'ester d'acide stéarique choisi dans le groupe comprenant le sorbitan stearate, le PEG-20 methylglucose sesquistearate, le methylglucose sesquistearate, le sucrose stearate, le sucrose tristearate, le glyceryl stearate, le PEG-20 stearate, le glycol distearate, le glyceryl stearate citrate, le polyglyceryl stearate, le polyglyceryl distearate, le polyglyceryl diisostearate et leurs dérivés, ou un quelconque mélange de ceux-ci.

Dans la présente par « dérivés d'esters d'acide phosphorique » on entend tout dérivé d'ester d'acide phosphorique connu de l'homme du métier et/ou disponible dans le commerce susceptible d'être utilisé dans une base cosmétique. Il peut s'agir par exemple un dérivé choisi dans le groupe comprenant le potassium cétyl phosphate, le C9-15 Alkyl Phosphate, le C20-22 Alkyl Phosphate, le stéaryle phosphate ou un quelconque mélange de ceux-ci.

Dans la présente par « ester d'acide citrique » on entend tout ester d'acide citrique connu de l'homme du métier et/ou disponible dans le commerce susceptible d'être utilisé dans une base cosmétique. Il peut s'agir par exemple un ester d'acide citrique choisi dans le groupe comprenant le glyceryl oleate citrate, glyceryl stéarate citrate, glyceryl citrate/lactate/linoleate/oleate, le glyceryl cocoate/citrate/lactate, polyglyceryl-3 dicitrate/stearate, le polyglyceryl-3 sunflowerseedate/Citrate crosspolymer, ou un quelconque mélange de ceux-ci.

Dans la présente par « ester de polyéthylène glycol » on entend tout ester de polyéthylène glycol connu de l'homme du métier et/ou disponible dans le commerce susceptible d'être utilisé dans une base cosmétique. Il peut s'agir par exemple un ester de polyéthylène glycol choisi dans le groupe comprenant le PEG-100 stearate, le PEG-20 methylglucose sesquistearate, le PEG-20 glyceryl stearate, le PEG-8 cire d'abeille (« beeswax »), le tribehenin PEG-20 esters, ou un quelconque mélange de ceux-ci.

Dans la présente par « éther de polyéthylène glycol » on entend tout éther de polyéthylène glycol connu de l'homme du métier et/ou disponible dans le commerce susceptible d'être utilisé dans une base cosmétique. Il peut s'agir par exemple d'un éther de polyéthylène glycol choisi dans le groupe comprenant le ceteareth-20, le ceteareth-12, le ceteth-20, le steareth-20 ou un quelconque mélange de ceux-ci.

Dans la présente par dérivé de « cires d'origine végétale » on entend tout dérivé de cires d'origine végétale connu de l'homme du métier et/ou disponible dans le commerce susceptible d'être utilisé dans une base cosmétique. Il peut s'agir par exemple d'un dérivé de cires d'origine végétale choisi dans le groupe comprenant une cire de jojoba, une cire de carnauba, une cire de riz, une cire de soja, une cire de candelilla, une cire de fleurs, par exemple une cire de fleurs de jasmin, cire de fleurs de lavande, une cire de fruits ou un quelconque mélange de ceux-ci.

Dans la présente par « dérivé de cires d'origine animale » on entend tout dérivé de cires d'origine animale connu de l'homme du métier et/ou disponible dans le commerce susceptible d'être utilisé dans une base cosmétique. Il peut s'agir par exemple de cires d'abeilles.

Dans la présente, la base concentrée peut comprendre au moins un, au moins deux, au moins trois émulsionnant(s).

Dans la présente, l'émulsionnant peut être présent dans la base de formulation cosmétique à une concentration comprise de 2 à 10% en poids par rapport au poids total de ladite base de formulation cosmétique, par exemple de 3 à 7,5% en poids par rapport au poids total de ladite base de formulation.

Avantageusement, l'émulsionnant peut être choisi dans le groupe comprenant au moins un ester de polyglycérol, un alcool gras, un sucro-ester ou un quelconque mélange de ceux-ci. Il peut s'agir par exemple d'un émulsionnant choisi dans le groupe comprenant le polyglyceryl-3-beeswax, l'alcool cetylique, le polyglyceryl-6-distearate, des esters jojoba, du sucrose laurate, du sucrose stéarate ou un quelconque mélange de ceux-ci.

Avantageusement, lorsque l'émulsionnant est choisi dans le groupe comprenant le polyglyceryl-3-beeswax, l'alcool cetylique, le polyglyceryl-6-distearate, des esters jojoba ou un quelconque mélange de ceux-ci, il peut être présent dans la base de formulation cosmétique à une concentration comprise entre 5 et 8% en poids par rapport au poids total de ladite base de formulation cosmétique.

Avantageusement, lorsque l'émulsionnant est choisi dans le groupe comprenant du sucrose laurate, du sucrose stéarate ou un quelconque mélange de ceux-ci. Il peut être présent dans la base de formulation cosmétique à une concentration comprise entre 2 et 4% en poids par rapport au poids total de ladite base de formulation cosmétique

Dans la présente, la base concentrée peut comprendre en outre au moins un agent de toucher.

Dans la présente ledit au moins agent de toucher peut être tout agent de toucher connu de l'homme du métier et/ou disponible dans le commerce susceptible d'être utilisé dans une base cosmétique. Il peut s'agir par exemple d'un agent de toucher choisi dans le groupe comprenant des charges organiques, par exemple des poudres de polysaccharides, par exemple des poudres d'amidon natif, par exemple de maïs, de riz, de tapioca et/ou de blé, d'amidon modifié et de cellulose, des poudres de polymères acryliques, par exemple le poly(méthacrylate de méthyle), de polyamides et/ou de polyoléfines, des poudres d'algues séchées, par exemple des poudres de Corallina officinalis; des charges inorganiques, par exemple de la silice, des argiles, de la perlite et/ou du talc, ou un quelconque mélange de ceux-ci.

Avantageusement, ledit au moins agent de toucher peut être choisi dans le groupe comprenant de l'amidon de maïs, l'amidon de riz, de l'amidon de tapioca, de l'amidon de blé, la silice et le poly(méthacrylate de méthyle).

Dans la présente, l'agent de toucher peut être présent dans la base de formulation cosmétique à une concentration comprise de 1 à 6% en poids par rapport au poids total de ladite base de formulation cosmétique, par exemple de 2 à 4% en poids par rapport au poids total de ladite base de formulation cosmétique.

Avantageusement, l'agent de toucher peut permettre d'obtenir des textures de composition comprenant la base cosmétique concentrée adaptées et/ou modulées en fonction de l'application cosmétique envisagée.

Dans la présente, la base concentrée de formulation cosmétique peut comprendre un ou plusieurs adjuvants connus de l'homme du métier, de préférence à l'exception de tensioactifs sulfatés. Il peut s'agir par exemple d'un ou plusieurs adjuvant(s) choisi(s) parmi des agents de type tensioactif amphotère, des agents hydratants, des agents émollients, des composés siliconés, des parfums, des conservateurs, des céramides, et pseudo-céramides, des vitamines et les provitamines, des protéines, des agents séquestrants, des agents alcanisants, des agents acidifiants, des agents réducteurs, des agents anti-oxydants, des colorants ou tout autre adjuvant, de préférence à l'exception de tensioactifs.

La base concentrée de formulation cosmétique selon l'invention peut être utilisée comme base de composition cosmétique.

La présente invention a donc également pour objet une composition cosmétique comprenant une base concentrée de formulation cosmétique selon l'invention.

Dans la présente, la composition cosmétique comprenant une base concentrée selon l'invention, peut être obtenue par mélange/dilution à froid de la base concentrée de formulation cosmétique avec au moins une solution.

Dans la présente l'étape de mélange peut être réalisée sous agitation, par exemple sous agitation manuelle, sous agitation mécanique avec un outillage laboratoire, pilote ou industriel.

Dans la présente l'étape de mélange peut être réalisée à froid, par exemple sans chauffage de la base cosmétique concentrée et/ou de la solution. Par exemple, l'étape de mélange peut être réalisée à une température comprise de 5 à 40 °C, de 20 à 30°C.

Dans la présente, la solution peut être toute solution connue de l'homme du métier et/ou disponible dans le commerce susceptible d'être mélangée avec une base cosmétique. Il peut s'agir par exemple d'une solution aqueuse, une solution huileuse, une solution hydroalcoolique, une solution hydroglycérinée. Il peut s'agir également d'une solution épaisse, par exemple une solution sous la forme de purée, par exemple une purée de végétaux, par exemple de fleurs, de fruits. Il peut s'agir par exemple d'une solution sous forme de poudre, par exemple un extrait de végétal sous forme de poudre. Il peut s'agir également d'une solution sous la forme d'une émulsion, par exemple une émulsion huile dans l'eau comprenant par exemple au moins un actif cosmétique, par exemple caféine. Il peut s'agir également par exemple d'eau, de solvant comprenant au moins un actif cosmétique et/ou d'extrait comprenant au moins un actif cosmétique, quelque soit sa forme. Il peut s'agir par exemple de toute solution adaptée pour son incorporation dans un produit cosmétique. Il peut s'agir par exemple d'une huile essentielle, d'un extrait alcoolique, d'un extrait aqueux, d'un extrait huileux de plantes, d'une purée végétale, d'une composition aqueuse comprenant au moins un actif cosmétique, d'une composition huileuse comprenant au moins un actif cosmétique, d'une composition alcoolique comprenant au moins un actif cosmétique, d'une composition sous forme émulsionnée ou pulvérulente.

Dans la présente, la base concentrée de formulation cosmétique peut être présente dans la composition cosmétique obtenue à une concentration de 30 à 80% en poids, par exemple de 40 à 70% en poids par rapport au poids total de ladite composition cosmétique.

Dans la présente par actifs cosmétiques on entend tout actif cosmétique connu de l'homme du métier et/ou disponible dans le commerce. Il peut s'agir par exemple d'un actif cosmétique cutané et/ou capillaire. Il peut s'agir par exemple d'un actif cosmétique obtenu par synthèse chimique, par exemple l'ascorbyl glucoside, un actif cosmétique naturel, par exemple un extrait d'origine végétale, par exemple à partir de toute partie de plantes, de plantes entières, de fleurs, de fruits, d'un extrait d'origine animale, par exemple du miel et/ou un extrait issu d'un procédé biotechnologique. Il peut s'agir par exemple d'un actif cosmétique choisi parmi un agent anti-âge, un agent hydratant, un agent apaisant, un agent agissant sur la microcirculation, un agent raffermissant, un actif minceur, un agent tenseur, un agent éclaircissant, un agent blanchissant, un filtre solaire, un actif cosmétique détergent, un actif déodorant, un actif maquillage ou de toucher, un colorant capillaire ou un mélange de ces agents. Ces agents peuvent être ceux couramment utilisés dans les produits cosmétiques et/ou dermatologiques. Des exemples d'agents anti-âge utilisables peuvent être par exemple le silicium ou la vitamine C. Des exemples d'agents hydratants utilisables peuvent être par exemple la glycérine, l'acide hyaluronique ou le D-Panthenol. Des exemples d'agents apaisants utilisables peuvent être par exemple le bisabolol ou l'allantoïne. Des exemples d'agents agissant sur la microcirculation peuvent être par exemple l'escine. Des exemples d'agents raffermissants peuvent être par exemple un dérivé de silicium, la caféine, des agents tenseurs par exemple des protéines d'amande. Des exemples d'agents blanchissants peuvent être par exemple des inhibiteurs de tyrosinase, des inhibiteurs de la mélanine, il peut s'agir par exemple de la vitamine C, des extraits végétaux, par exemple des extraits de fleurs, de racines, par exemple des extraits de fleurs de reine des prés, par exemple de Spirae ulmaria, des extrait de racines de murier, par exemple de Morus alba. Des exemples d'actifs minceurs peuvent être, par exemple, la caféine, un extrait d'amande.

La composition cosmétique selon l'invention peut comprendre une concentration en poids de principe actifs cosmétiques de 0,001 à 70%, par exemple de 5 à 50%, de 10 à 30% en poids d'actifs cosmétiques par rapport au poids total de la composition cosmétique.

Dans la présente, le rapport entre la base cosmétique et les actifs cosmétique présents dans la composition cosmétique peut être compris de 0,5 à 4, par exemple de 1 à 2,5.

La présente invention se rapporte également à une composition cosmétique comprenant une base de formulation cosmétique concentrée selon l'invention, et un ou plusieurs principe(s) actif(s).

Dans la présente, par composition cosmétique, on entend toute composition cosmétique connue de l'homme du métier. Il peut s'agir par exemple d'une composition topique, une composition capillaire. La composition cosmétique ou dermatologique peut être, par exemple une composition pour le soin du visage, du corps, des cheveux, par exemple des compositions pour le visage et/ou le corps et/ou les cheveux.

Avantageusement, la base concentrée de formulation cosmétique et/ou la composition cosmétique comprenant la base concentrée de formulation cosmétique selon l'invention a une stabilité et une viscosité adaptées à toute utilisation cosmétique.

Avantageusement, la base concentrée de formulation cosmétique peut être une émulsion concentrée stable et homogène comprenant une faible teneur en eau, notamment une teneur en eau très inférieure aux bases de formulations connues. En outre, les inventeurs ont démontré de manière surprenante que la base de formulation selon l'invention est une base « prête à l'emploi » qui peut avantageusement être diluée/mélangée avec une solution pour la formation d'une composition cosmétique, par exemple via un simple mélange, par exemple « à la main » ou avec un agitateur mécanique.

Les inventeurs ont démontré que la base concentrée de formulation cosmétique peut être avantageusement conservée dans le temps sans détérioration de ses propriétés physicochimiques et/ou microbiologiques.

En outre, les inventeurs ont démontré avantageusement que la base concentrée de formulation cosmétique peut permettre l'obtention de compositions cosmétiques juste avant sa commercialisation et/ou l'obtention de compositions cosmétiques « à la maison » par simple mélange par le consommateur. Aussi, la base concentrée de formulation cosmétique permet avantageusement l'obtention de compositions cosmétiques « fraiches » et/ou « instantanées »quelque soit le temps de conservation de la base.

La présente invention se rapporte également à l'utilisation cosmétique d'une composition cosmétique comprenant une base concentrée de formulation cosmétique selon l'invention

Dans la présente, l'utilisation peut être mise en oeuvre par application de la composition sur la peau ou les phanères.

Il peut s'agir, par exemple d'une simple application sur la peau ou les phanères ou d'une application accompagnée d'un massage ou encore d'une application en couches épaisses avec un temps de pose, par exemple de 1 à 10 minutes avant pénétration ou rinçage.

L'application est de préférence réalisée avec une quantité suffisante de la composition afin que toute la surface de la peau ou des phanères à traiter soit traitée. Il peut s'agir par exemple d'une application classique, Il peut s'agir par exemple d'une application classique, comme une crème sur la peau. Il peut s'agir par exemple aussi d'une application pour former un masque traitant.

L'application peut être toute application souhaitée par l'utilisateur/l'utilisatrice, par exemple une application quotidienne, biquotidienne, hebdomadaire et/ou bi-mensuelle. Il peut s'agir par exemple d'une application une fois par semaine, deux fois par semaine, trois fois par semaine ou plus, une fois par jour, deux fois par jour ou plus.

D'autres avantages pourront encore apparaître à l'homme du métier à la lecture des exemples ci-dessous illustrés par les figures annexées, données à titre illustratif.

### Brève description des figures

- La figure 1 est une photographie au microscope d'une base concentrée de formulation cosmétique après étalement sur une lame
- La figure 2 est une photographie au microscope d'une base concentrée de formulation cosmétique après mélange avec une solution et étalement sur une lame

### EXEMPLES

### Exemple 1 : Exemple de base concentrée de formulation cosmétique

Les compositions précitées ont été préparées selon le procédé suivant en utilisant pour les étapes de mélange : Rayneri /défloculeuse, comme contenant un bécher de 600 ml pour la réalisation de 400 gr de produit, une plaque chauffante, des spatules, des maryses et une balance.

Les ingrédients utilisés pour la fabrication de bases concentrées de formulations cosmétiques sont décrits dans le tableau 1 ci-dessous.

**Tableau 1 : ingrédients pour la fabrication de bases concentrées de formulations cosmétiques**

| Phases | INGREDIENTS | Base 1 (Pourcentage en poids par rapport au poids total de la composition) | Base 2 (Pourcentage en poids par rapport au poids total de la composition) | Base 3 (Pourcentage en poids par rapport au poids total de la composition) |
|---|---|---|---|---|
| A | Eau | QSP 100 | QSP 100 | 5 |
| | Glycérine | 21 | 5-30 | 26 |
| | Gomme végétale | 0,1 | 0,1-1 | |
| | Ester de sucres | - | - | 3,75 |
| | Gomme issue de la biotechnologie | 0,2 | 0,3 | - |
| B | Alcool gras | 0,7 | 0,7 | - |
| | Beurre végétal | 2,9 | 2,9 | - |
| | Emollient | 21,5 | 21,5 | QSP 100 |
| | Ester de polyglycérol issu du riz | - | 5,7 | - |
| | Cire émulsionnante | 5,7 | - | - |
| C | Agent de toucher | 2,9 | 1,4 | - |
| | Agent filmogène | 0,6 | - | |
| | Ajusteur de pH | QSP | QSP | |
| | Gélifiants acrylates | - | - | 1,5 |
| | Emollient | - | - | 5 |
| D | Agent de toucher | - | - | 4 |

Procédé de fabrication pour les bases 1 et 2: Les matières premières de la phase A ont été pesées à l'aide de la balance et chauffées à 75°C en utilisant le Rayneri/Défloculeuse et la plaque chauffante.

Les ingrédients de la phase B ont été pesés à l'aide de la balance et chauffés à 75°C (bécher/plaque chauffante).

Lorsque les températures des phases A et B ont été atteintes, la phase B a été ajoutée dans la phase A sous Rayneri afin de réaliser une émulsion et la vitesse a été augmentée progressivement à 3000 tr/min. le mélange a été laissé émulsionné à cette vitesse pendant 3 min puis diminué à 800 tr/min. L'ensemble a été refroidi à une vitesse de 800 tr/min. Une fois la température de 45°C atteinte, les ingrédients de la phase C ont été ajoutés et la vitesse a été raugmentée à 1500 tr/min pendant 3 min. L'ensemble a été laissé refroidi à 30°C à 800 tr/min.

Procédé de fabrication pour la base 3: la glycérine a été pesée dans un bécher de 600ml et chauffées à 80°C en utilisant une plaque chauffante. Les esters de sucre, à savoir un mélange de sucrose laurate et sucrose stéarate ont été pesés et incorporés dans la glycérine sous agitation à 150 tours par minutes à l'aide d'une Rayneri/Défloculeuse. Après la fonte des esters de sucres, l'eau a été ajoutée et l'ensemble mis sous agitation à une vitesse de 200 tr/min et chauffage jusqu'à atteindre une température de 80°C.

Lorsque la température de 80°C a été atteinte, les ingrédients de la phase B ont été ajoutés dans la phase A sous Rayneri afin de réaliser une émulsion et la vitesse a été augmentée progressivement à 3000 tr/min. le mélange a été laissé émulsionné à cette vitesse pendant 2 min puis diminué à 800 tr/min. Les gélifiants acrylates, à savoir un mélange de sodium polyacrylate et de sodium acryloyldimethyltaurate copolymer ont été mouillés dans les 5% d'émollients puis incorporés dans le mélange sous agitation à une vitesse de 800 tr/min. L'ensemble a été refroidi jusqu'à 30°C sous agitation à 800 tr/min.

Les bases obtenues étaient stables et homogènes et correspondaient à des émulsions huiles dans l'eau.

### Exemple 2 : Exemple de compositions cosmétiques comprenant une base concentrée de formulation cosmétique

Dans cet exemple, quatre compositions cosmétiques comprenant une base concentrée de formulation cosmétique ont été préparées à partir des bases décrites dans l'exemple 1 ci-dessus.

A partir de la base concentrée de formulation cosmétique n°2, une solution comprenant des actifs cosmétiques, à savoir une purée végétale issue de framboises et de roses a été ajoutée à froid, c'est-à-dire la base de crème a été diluée par l'ajout de la purée par simple agitation en mélangeant 3 min à 1500 tr/min.

La composition cosmétique obtenue est décrite dans le tableau 2 suivant :

**Tableau 2 : compositions cosmétique**

| INGREDIENTS | Pourcentage en poids par rapport au poids total de la composition |
|---|---|
| Eau | QSP 100 |
| Glycérine | 15 |
| Gomme végétale | 0,7 |
| Gomme issue de la biotechnologie | 0,14 |
| Alcool gras | 0,5 |
| Beurre végétal | 2 |
| Emollient | 15 |
| Ester de polyglycérol issu du riz | 4 |
| Agent de toucher | 2 |
| Agent filmogène | 0,4 |
| Ajusteur de pH | QSP |
| Purée végétale | 30 |

Tel que démontré, la base concentrée permet avantageusement d'obtenir des compositions cosmétiques avec une forte quantité d'une solution comprenant des actifs cosmétiques, ici la purée végétale. En outre, la composition obtenue était stable et présentait une viscosité de crème, permettant par exemple son conditionnement dans un pot.

En outre, deux autres compositions ont été réalisées en incorporant respectivement 40 et 50 % en poids par rapport au poids total de la composition cosmétique de purée végétale.

Ces compositions étaient également stables et présentaient également une viscosité de crème, permettant notamment leurs conditionnements en pots.

En outre, la dilution de la base concentrée par des concentrations croissantes en solution comprenant au moins un actif cosmétique diminue la teneur en eau initiale de la base concentrée de formulation cosmétique. Aussi, une évaluation de la stabilité physicochimique et microbiologique a été effectuée.

Pour ce faire une mesure de l'Aw représentant la disponibilité de l'eau dans un milieu a été effectuée. La mesure de l'Aw prend en compte les interactions physico-chimiques entre l'eau et les autres constituants du milieu. La mesure d'Aw d'un produit se fait par mesure de l'humidité relative de l'air en équilibre avec ce produit. L'humidité relative HR est mesurée en % selon la formule suivante

Aw= HR/100 HR = (p/po )x 100 avec p= pression partielle de l'eau dans l'aire et po = pression partielle de l'eau dans un air saturé en eau.

La mesure de l'Aw a été réalisée avec un Awmètre de marque Novasina et de modèle Labtouch.

Une évaluation de la stabilité a été réalisée selon le protocole suivant : chaque base a été conditionnée dans 5 piluliers de 50 ml qui ont été stockés à 4 températures différentes à savoir 4°C, température ambiante : 20°C, à 40°C, et 50°C. La stabilité du produit stocké à 4°C, température ambiante : 20°C et à 40°C, a été évaluée pendant 3 mois. Celle de bases stockés à 50°C a été évaluée pendant 1 mois. Un contrôle des piluliers stockés à 50°C a été réalisé toutes les semaines sur le mois. Un contrôle des piluliers stockés à 4°C, à 20°C et à 40°C a été réalisé toutes les semaines pendant un mois puis tous les 15 jours jusqu'à 3 mois de stabilité.

Les résultats sont présentés dans le tableau 3 ci-dessous :

| **Compositions** | **1** | **2** | **3** |
|---|---|---|---|
| Teneur en eau (% en poids par rapport au poids total) | 39 | 29,82 | 15,78 |
| Aw de la base concentrée | 0,857 | 0,784 | 0,615 |
| Résultats vieillissement de la base concentré | Stable | Stable | stable |
| % en purée (% en poids par rapport au poids total) | 30 | 40 | 50 |
| Résultats de stabilité de la composition comprenant la base mélange base concentrée + purée | Stable et homogène | Stable et homogène | Stable et homogène |

Tel que démontré ci-dessus, les compositions obtenues sont stables tant au niveau microbiologique que physicochimique. En outre, la base concentrée de formulation cosmétique permet d'obtenir des compositions cosmétiques stables comprenant une quantité importante d'actifs cosmétiques.

Par ailleurs, à partir de la base concentrée n°3 de l'exemple 1 ci-dessus, une composition cosmétique a été obtenue par mélange de la base avec une eau florale de rose par dilution 50/50 et agitation.

Le tableau 4 résume les caractéristiques de la composition cosmétique obtenue.

**Tableau 4 : Composition cosmétique**

| INGREDIENTS | Pourcentage en poids par rapport au poids total de la composition |
|---|---|
| Eau | 2,5 |
| Glycérine | 13 |
| Esters de sucre | 1,875 |
| Emollient | 27,345 |
| Gélifiants acrylates | 0,75 |
| Emollient | 2,5 |
| Agent de toucher | 2 |
| Eau florale de rose | 50 |

Une évaluation de la base concentrée de formulation utilisée et de composition cosmétique obtenue a été effectuée par photographie au microscope (Leica modèle ICCS50) sous grossissement x10. Pour ce faire, un point de crème a été déposé et mis sous lame.

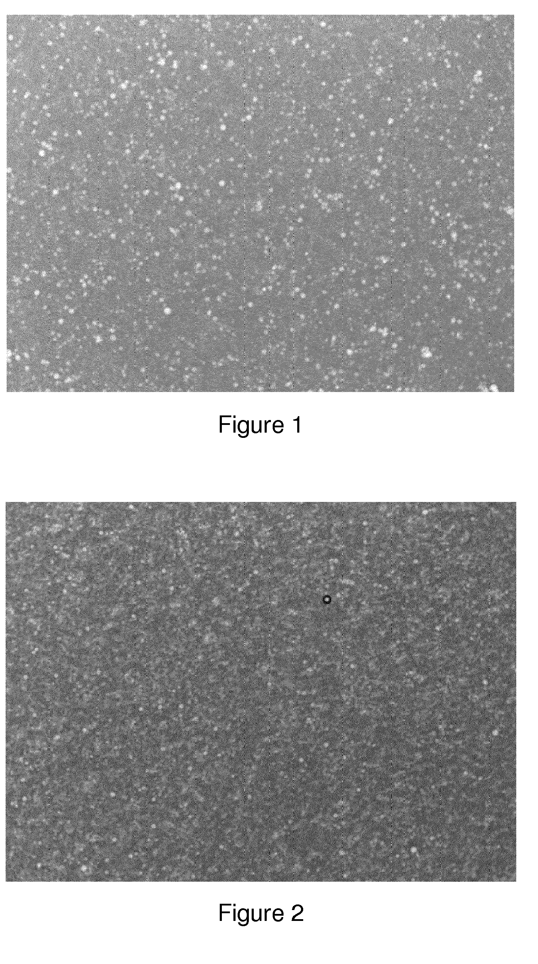

La figure 1 est une photo de la base concentrée avant mélange. Tel que montré sur cette photo, un tapis fin et homogène a été observé et est garant d'une bonne stabilité de la base concentrée malgré la quantité d'eau réduite présent dans la base. La figure 2 est une photo de la base concentrée après mélange avec l'eau florale. Tel que montré sur cette photo, un tapis fin et homogène a été également observé et est garant d'une bonne stabilité de la composition obtenue.

Des résultats similaires ont été obtenus en incorporant dans la base de l'eau osmosée à une dilution 50/50.

Tel que démontré ci-dessous, la base concentrée de formulation est stable dans le temps tant au niveau physicochimique que microbiologique. En particulier, les inventeurs ont clairement démontré que la base selon l'invention comprenant une teneur en eau faible, avantageusement inférieure à une valeur de Aw inférieure à 0,6 est stable dans le temps tant au niveau physicochimique que microbiologique. En outre, la base concentrée de formulation permet avantageusement l'obtention de compositions cosmétiques stables et homogènes par simple mélange à température ambiante, par exemple 20°C, de la base avec une solution comprenant au moins un actif cosmétique.

Aussi, la base concentrée de formulation cosmétique permet avantageusement l'obtention de composition cosmétique par « simple » dilution de ladite base et l'obtention de crème cosmétique « fraiche » ou « fabriquée à la maison ».

De plus, tel que démontré ci-dessus, la base concentrée de formulation cosmétique est microbiologiquement stable et ne nécessite pas la présence de conservateur. En particulier, les inventeurs ont clairement démontré que la base selon l'invention comprenant une teneur en eau faible, avantageusement inférieure à une valeur de Aw inférieure à 0,6, est stable dans le temps ne nécessite pas la présence de conservateur. Aussi, la base concentrée de formulation cosmétique permet avantageusement l'obtention de compositions cosmétiques sans conservateur tout en conservant une forte stabilité microbienne due notamment à sa faible Aw (peu d'eau et riche en glycérine).

L'absence de conservateur dans les produits cosmétiques permet avantageusement de réduire les éventuels phénomènes d'intolérance cutanée tout en ayant un temps de conservation similaire aux compositions cosmétiques comprenant des conservateurs.

De plus, de part la stabilité physicochimique et microbiologique de la base concentrée de formulation, celle-ci peut avantageusement être conservée et stockée sans altération.

En outre, la base concentrée de formulation permet en fonction de la quantité de solution ajoutée/mélangée d'obtenir des compositions cosmétiques avec des textures et/ou sensorialités différentes et toujours adaptée pour une utilisation cosmétique.

Enfin, l'absence de chauffage lors du mélange de la base concentrée de formulation cosmétique permet avantageusement la conservation d'actifs cosmétiques thermosensibles dans la composition cosmétique obtenue. En d'autres termes, la base concentrée de formulation cosmétique permet l'obtention de compositions cosmétiques sans dégradation d'actifs cosmétiques notamment thermosensibles.

## Revendications

1. Base de formulation concentrée cosmétique comprenant :
- une phase aqueuse glycérinée comprenant de 15 à 50 % en poids de glycérine, de 2 à 50% en poids d'eau, et de 0,2 à 3% en poids d'au moins un gélifiant par rapport au poids total de la base de formulation,
- une phase huileuse comprenant de 20 à 60% en poids d'au moins un émollient, de 2 à 10% en poids d'au moins un émulsionnant par rapport au poids total de la base de formulation.

2. Base concentrée selon la revendication 1, dans laquelle ledit au moins un gélifiant est choisi dans le groupe comprenant les silicates, les celluloses, les acrylates, les dérivés d'algues, les gommes végétales, les pectines, les dérivés d'amidon, la gomme de xanthane, la gomme de sclérotium, une gomme d'origine naturelle ou un mélange de ceux-ci.

3. Base concentrée selon la revendication 1, dans laquelle ledit au moins un gélifiant est choisi dans le groupe comprenant la bentonite, l'hectorite, la montmorillonite, le sodium magnésium silicate, la cellulose microcrystalline, la carboxyméthyle cellulose, la gomme de xanthane, la gomme de sclerotium, l'acrylates/Beheneth-25 méthacrylate copolymer, le copolymère acrylates/C₁₀₋₃₀ alkyl acrylate, sodium polyacrylate starch, l'hydroxyéthyle acrylate / sodium acryloyldiméthyle taurate copolymère, les carbomers, la gomme de caroube, la gomme guar, la gomme de tara, la gomme d'acacia du Sénégal, les pectines, les caraghénanes, l'agar, le sodium carboxymethyl gomme naturelle d'amidon, l'hydroxypropyl phosphate d'amidon ou un mélange de ceux-ci.

4. Base concentrée selon l'une quelconque des revendications 1 à 3, dans laquelle ledit au moins un émulsionnant est choisi dans le groupe comprenant monoester ou polyester de glycérol, les dérivés de lécithine, les émulsionnants issus de la condensation d'un alcool gras et d'un sucre choisi dans le groupe comprenant le sorbitol, glucose, saccharose, les esters de sorbitol, les esters de glucose, les esters de saccharose, les alcools gras, des acides gras, les dérivés d'acides aminés, les dérivés d'esters d'acide laurique, les dérivés d'esters d'acides stéariques, les dérivés d'esters d'acides phosphoriques, des esters d'acide citrique, esters de polyéthylène glycol, des éthers de polyéthylène glycol, des sarcosinates, des dérivés de cires d'origine végétale ou animale.

5. Base concentrée selon l'une quelconque des revendications 1 à 4, dans laquelle l'émollient est choisi dans le groupe comprenant les huiles végétales, les beurres végétaux, les esters d'origine synthétique ou d'origine végétale ou un mélange de ceux-ci.

6. Base concentrée selon l'une quelconque des revendications précédentes, ladite base étant une émulsion.

7. Base concentrée selon l'une quelconque des revendications précédentes, le pH de ladite base étant compris de 4 à 10.

8. Composition cosmétique comprenant une base concentrée selon l'une quelconque des revendications 1 à 7.

9. Composition cosmétique selon la revendication 8, dans laquelle la base de formulation cosmétique est présente dans la composition cosmétique à une concentration de 30 à 80% en poids par rapport au poids total de ladite composition cosmétique.

10. Composition cosmétique selon la revendication 8 ou 9, dans laquelle la composition comprend de 20 à 70 % en poids d'actifs cosmétiques par rapport au poids total de ladite composition cosmétique.

11. Composition cosmétique selon l'une quelconque des revendications 8 à 10, dans laquelle le rapport entre la base cosmétique et les actifs cosmétique est compris de 0,5 à 4.

12. Utilisation cosmétique d'une composition selon l'une quelconque des revendications 8 à 11.
